# EUROPÄISCHE PATENTSCHRIFT

(11) **EP 1 018 926 B1**
(45) Veröffentlichungstag und Bekanntmachung des Hinweises auf die Patenterteilung: **03.03.2004**
(21) Anmeldenummer: 98952672.8
(22) Anmeldetag: 30.09.1998
(51) Int. Cl.: A61B 1/00, A61B 1/12

(54) **ABSCHLUSSELEMENT FÜR ENDOSKOP**
Closing Element for an Endoscope
ELEMENT DE FERMETURE POUR ENDOSCOPE

(30) Priorität: 01.10.1997 DE 19743431
(43) Veröffentlichungstag der Anmeldung: 19.07.2000
(73) Patentinhaber: Karl Storz GmbH & Co. KG, 78532 Tuttlingen (DE)
(72) Erfinder: KEHR, Ulrich, D-73760 Ostfildern (DE); RUDISCHHAUSER, Jürgen, D-78532 Tuttlingen (DE); IRION, Klaus, M., D-78576 Liptingen (DE)
(74) Vertreter: Weller, Wolfgang, Dr.
(86) Internationale Anmeldenummer: PCT/EP1998/006207
(87) Internationale Veröffentlichungsnummer: WO 1999/016341

(56) Entgegenhaltungen:
- DE-A- 3 708 124
- DE-A- 4 211 547
- DE-A- 4 311 577
- DE-A- 4 341 062
- US-A- 4 279 246
- US-A- 5 369 525
- US-A- 5 402 768

## Beschreibung

Die Erfindung betrifft ein Endoskop, mit einem Kopf am proximalen Ende, mit einem mit dem Kopf verbundenen Außenrohr, mit einem im Außenrohr aufgenommenen Innenrohr, in welchem optische Bauelemente aufgenommen sind, wobei am distalen Ende des Innenrohrs ein Fenster aus lichtdurchlässigem Material angeordnet ist, sowie mit Lichtleitern, die in einem Zwischenraum zwischen Innenrohr und Außenrohr aufgenommen und axial bis zum distalen Ende des Zwischenraumes geführt sind, wobei ein Abschlußelement aus lichtdurchlässigem Material vorgesehen ist, das das distale Ende des Außenrohrs, zumindest im Bereich des Innenrohres und des die Lichtleiter aufnehmenden Zwischenraumes, hermetisch dicht abschließt, daß das Abschlußelement im Bereich des distalen Endes des Innenrohrs das Fenster aufweist, über das Licht in das Innenrohr eintreten kann, und daß das Fenster umfänglich von einer lichtundurchlässigen Schicht umgeben ist, die das Fenster radial vor Eintritt von Beleuchtungslicht abschirmt, das, von den Lichtleitern kommend, durch das Abschlußelement hindurchgeführt wird und am distalen Ende austritt.

Ein derartiges Endoskop ist aus der DE 42 11 547 A bekannt.

Aus der US-A-5 402 768 ist ein einteiliges Abschlußelement bekannt, welches lediglich mit seiner äußeren Umfangskante mit dem Außenrohr verbunden ist.

Aus der eingangs genannten DE 42 11 547 A ist eine Schutzabdeckung für das distale Ende von Endoskopen bekannt, das ein erstes Bauteil aufweist, das eine an das Ende des Innenrohrs, das die optischen Bauelemente aufnimmt, ansetzbare Fassung aufweist. Die Fassung selbst trägt mittig ein Fenster mit optischer Wirkung, über das von der Außenseite Licht in das optische System eintreten kann.

Distalseitig steht das Fenster über die Fassung vor und über den vorstehenden Bereich ist ein zweites Teil geschoben, das die verbleibende Endfläche des distalen Endes des Endoskops abdeckt, wobei dieses Teil mit dem äußeren Ende des Außenrohrs verbunden ist.

Um einen Übertritt von Beleuchtungslicht in Querrichtung zu verhindern, ist zumindest an den Berührungsflächen zwischen der Außenseite des von der Fassung vorstehenden Fensters und der Innenseite des zweiten Teils eine lichtundurchlässige Schicht vorgesehen.

Um einen hermetisch dichtenden Abschluß sicherzustellen, muß für eine Abdichtung zwischen dem distalen Ende des Innenrohrs und der Fassung gesorgt werden, außerdem für einen hermetisch dichtenden Abschluß zwischen der Fassung und dem in die Fassung eingesetzten Fenster und zusätzlich für einen hermetisch dichtenden Abschluß zwischen dem äußeren abdeckenden Teil und dem darin aufgenommenen Fenster sowie zwischen dem äußeren Teil und dem ringförmigen, stirnförmigen Ende des Außenrohrs. Es treffen nun in diesem kritischen Dichtungsbereich mehrere unterschiedliche Materialien aufeinander, nämlich einerseits die Saphirschutzabdeckung, das Material der Fassung und die Materialien von Innenrohr und Außenrohr. Da unterschiedliche Materialien auch unterschiedliche Ausdehnungskoeffizienten haben, können bei dieser Konstruktion systemimmanent im Dauerbetrieb Undichtigkeiten auftreten. Darüber hinaus ist die Schutzabdeckung aus zahlreichen exakt aufeinander abzustimmenden Teilen aufgebaut und diese muß sowohl mit dem Innenrohr als auch mit dem Außenrohr dichtend verbunden werden. Die Fassung, die ebenfalls lichtundurchlässig sein muß, da ansonsten in Querrichtung über die Fassung Licht von den Lichtleitern in das optische System eintreten könnte, da die Fassung direkt an den Lichtleitern anliegt, bedeckt einen erheblichen Querschnitt des distalen Endes des Endoskops, so daß dieser Querschnitt weder zum Lichtaustritt für die Lichtleiter noch zum Lichteintritt in den Bildleiter zur Verfügung steht.

Es ist Aufgabe der vorliegenden Erfindung, hier Abhilfe zu schaffen und einen Abschluß des distalen Endes von Innenrohr und Zwischenraum, in dem die Lichtleiter aufgenommen sind, zu finden, der auf Dauer dicht ist und der einfach zu bewerkstelligen ist.

Erfindungsgemäß wird die Aufgabe dadurch gelöst, daß das Abschlußelement aus lediglich zwei Bauelementen aufgebaut ist, nämlich aus dem mittigen Fenster, das in das Abschlußelement eingesetzt ist und mit diesem nur über die umfängliche lichtundurchlässige Schicht verbunden ist, und daß das Abschlußelement nur mit seiner äußeren Umfangskante mit dem Außenrohr verbunden ist.

Erfindungsgemäß ist nunmehr vorgesehen, im Gegensatz zum eingangs erwähnten Stand der Technik, nicht das Innenrohr, das die optischen Bauelemente trägt, hermetisch dicht abzuschließen, sondern das Außenrohr, und zwar sowohl im Querschnittsbereich des Innenrohrs also auch im Querschnittsbereich des die Lichtleiter aufnehmenden Zwischenraums. Der distale Abschluß des Zwischenraums, in dem die Lichtleiter aufgenommen sind, wird nicht mehr durch eine Kittstelle bewerkstelligt, sondern durch ein separates Abschlußelement. Das heißt, die Lichtleiter reichen nicht bis zur Außenseite, sondern nur bis zur inneren Seite des Abschlußelements und sind zur Außenseite hin von diesem abgedeckt. Aufgrund der Tatsache, daß das gesamte Abschlußelement aus lichtdurchlässigem Material ausgebildet ist, kann das von den Lichtleitern am distalen Ende austretende Beleuchtungslicht durch das Abschlußelement hindurch zur Außenseite austreten, und durch das Fenster in dem Abschlußelement ist es möglich, von der Außenseite her das Bild in die Innenseite des Innenrohrs eintreten zu lassen.

Die lichtundurchlässige Schicht, die das Fenster umfänglich umgibt, sorgt dafür, daß vom Abschlußelement kein durch dieses hindurchtretendes Beleuchtungslicht als Streulicht in den Innenraum des Innenrohrs eintritt. Die lichtundurchlässige Schicht verlängert quasi das Innenrohr und schirmt dieses radial vor Beleuchtungslicht ab. Das Material der lichtundurchlässigen Schicht kann beliebig ausgewählt werden, mit der Bedingung, daß ein dichter Abschluß zwischen Fenster und dieses umgebenden Element möglich ist.

Aufgrund der Tatsache, daß das Abschlußelement hermetisch dicht mit dem Außenrohr verbunden ist, ist es nicht mehr notwendig, daß das distale Ende des Innenrohrs ebenfalls fest mit dem Abschlußelement verbunden ist, da der Bereich, in dem das Innenrohr distalseitig mündet, und der Bereich, in dem die Lichtleiterfasern münden, hermetisch durch das Abschlußelement abgeschlossen ist. Es entfällt somit die Notwendigkeit einer zusätzlichen mechanisch festen Verbindung des Innenrohrs am distalen Ende mit dem Außenrohr neben einer weiteren Verbindung am proximalen Ende. Es ist möglich, das Innenrohr nur an einer Stelle mechanisch mit dem Außenrohr zu verbinden, wodurch thermische Längendifferenzen zwischen Innen- und Außenrohr keine mechanischen Längsspannungen mehr verursachen können.

Durch diese Ausgestaltung ist es möglich, die Innenräume des Innenrohrs und auch den Zwischenraum, in dem die Lichtleiter aufgenommen sind, auf Dauer hermetisch dicht zur Außenseite hin abzuschließen, so daß weder durch die Art der Abdichtung bzw. Verbindung am distalen Ende noch durch Längendifferenzen zwischen Innenrohr und Außenrohr Undichtigkeiten auftreten können.

Sind im Endoskopschaft nur das Innenrohr mit den optischen Bauelementen und der Zwischenraum vorhanden, in denen die Lichtleiter an das distale Ende geführt werden, so schließt das Abschlußelement den gesamten lichten Innenquerschnitt des den Endoskopschaft darstellenden Außenrohrs am distalen Ende ab. Sind im Schaft noch weitere Kanäle oder Durchtrittsöffnungen zum Durchführen von Instrumenten oder von Flüssigkeiten oder Gasen vorhanden, deckt das Abschlußelement den Bereich ab, der dem Innenrohr und der distalen Öffnung des Zwischenraumes entspricht, an dem die Lichtleiter distalseitig münden. Der dichte Abschluß mittels des einfach aufgebauten und einfach ausgestalteten Abschlußelements ist durch Verkleben oder Verlöten einfach und sicher zu bewerkstelligen. Das Abschlußelement trägt und hält das Fenster, so daß keine zusätzliche Fassung notwendig ist. Außerdem entfällt eine Verbindung zwischen Fenster und Innenrohr.

Somit wird die Aufgabe vollkommen gelöst.

In einer weiteren Ausgestaltung der Erfindung ist das Abschlußelement aus dem Fenster, einer dessen umfängliche Mantelfläche bedeckenden lichtundurchlässigen Schicht und einem dieses umgebende Element zusammengesetzt.

Diese Maßnahme hat den Vorteil, daß das Abschlußelement als Verbundelement ausgebildet ist, so daß dieses den jeweiligen Querschnittsgeometrien von Innenrohr und Zwischenraum, in dem die Lichtleiter aufgenommen sind, angepaßt werden kann und dementsprechend auch die lichtundurchlässige Schicht, um das Innenrohr vor einem radialen Übertritt von austretendem Beleuchtungslicht abzuschirmen, jeweils der Geometrie des Innenrohrs angepaßt ist. Die hermetisch dichte Verbindung zwischen Abschlußelement und Außenrohr bzw. die hermetisch dichte Verbindung zwischen Fenster und dem diesen umgebenden Element kann durch an sich bekannte Maßnahmen, bspw. Verlöten, stattfinden, wie das aus dem eingangs erwähnten Stand der Technik bei der hermetisch dichten Verbindung zwischen dem Fenster und dem Innenrohr durchgeführt worden ist.

Das das mittige Fenster umgebende Element kann je nach Geometrie und Anordnung von Innenrohr relativ zum Außenrohr variieren. Bei einer koaxialen Anordnung von Außenrohr und Innenrohr ist dieses als Ring ausgebildet, bei nicht koaxialer Anordnung als sichelartiges Gebilde, das auf jeden Fall den entsprechenden Raum, der nicht durch das Fenster abdeckt wird, lichtdurchlässig abdeckt, um einen Lichtaustritt des Beleuchtungslichtes zu ermöglichen. Ist das Innenrohr bspw. längs einer Mantellinie an der Innenseite des Außenrohrs gelegt, weist das Element im Querschnitt eine nicht vollständig geschlossene Form auf, d.h. in diesem Fall ist das Innenrohr nicht vollständig von Lichtleitern umrundet.

In einer weiteren Ausgestaltung der Erfindung ist die lichtundurchlässige Schicht derart ausgebildet, daß diese, neben der Abschirmung, auch für eine hermetisch dichte Verbindung zwischen Fenster und dieses umgebenden Element sorgt.

Diese Maßnahme hat den Vorteil, daß die lichtundurchlässige Schicht zwei Funktionen erfüllt, nämlich neben der Abschirmung auch als Verbindungsmaterial zwischen Fenster und dem diesem umgebenden Element dient.

In einer weiteren Ausgestaltung der Erfindung besteht die lichtundurchlässige Schicht aus einem Material, das einen Wärmeausdehnungskoeffizienten aufweist, der im Bereich der Wärmeausdehnungskoeffizienten der Materialien des Fensters und des dieses umgebenden Elements liegt.

Diese Maßnahme hat den erheblichen Vorteil, daß die Verbindungsstelle auch auf Dauer den hohen thermischen Beanspruchungen gewachsen ist.

In einer weiteren Ausgestaltung der Erfindung ist die lichtundurchlässige Schicht auf der umfänglichen Mantelfläche der Scheibe und/oder auf der radial inneren Mantelfläche des Ringelements aufgebracht.

Diese Maßnahme eröffnet nun die Möglichkeit, je nachdem, an welcher Stelle es am günstigsten ist, die Schicht aus lichtundurchlässigem Material aufzutragen. So kann es bspw. günstig sein, wenn das Fenster als kreisrunde Scheibe ausgebildet ist, die von einem stangenförmigen Material abgetrennt wird, deren umfängliche Außenseite mit der lichtundurchlässigen Schicht zu versehen, bzw. das stabförmige Material, von dem dieses abgelängt wird, zu beschichten, was bspw. durch Eintauchen eines Stabes in ein entsprechendes Medium einfach möglich ist.

Unter Umständen kann es auch günstiger sein, die radial innere Mantelfläche des die Scheibe umgebenden Elements mit der lichtundurchlässigen Schicht zu beschichten.

Insbesondere wenn das Material der lichtundurchlässigen Schicht auch zugleich für die dichte feste Verbindung dieser beiden Bauelemente sorgen soll, kann es günstig sein, beide Mantelflächen zu beschichten.

In einer weiteren Ausgestaltung der Erfindung besteht die lichtundurchlässige Schicht aus einem metallischen Material, das als vorgefertigte Überzugsschicht oder durch Sintern oder durch Aufdampfen auf die Mantelflächen aufgebracht ist.

Diese Maßnahmen haben nun den erheblichen Vorteil, daß, je nach konstruktiven Gegebenheiten, die lichtundurchlässige Schicht einfach und zuverlässig aufgebracht werden kann.

In einem einfach zu bewerkstelligenden Fall ist die lichtundurchlässige Schicht als metallisches Rohrstück ausgebildet, das auf die äußere Mantelfläche des Fensters passend aufgeschoben wird. Hat das metallische Material auch Eigenschaften eines Lötmaterials, kann dies dann zugleich zur Verbindung der beiden lichtdurchlässigen Bauelemente des Abschlußelements herangezogen werden, indem nämlich diese beiden miteinander verlötet werden.

Ein Aufdampfen oder ein Aufsintern von pulverförmigem Material wird dann vorteilhaft sein, wenn kompliziert geformte Mantelflächen beschichtet werden müssen. Dabei kann das Aufdampfen durch ein chemisches (CVD) oder ein physikalisches (PVD) Aufdampfverfahren erfolgen.

In einer weiteren Ausgestaltung der Erfindung ist auch die radial äußere Mantelfläche des das Fenster umgebenden Elements mit einem Material beschichtet, das eine hermetisch dichte Verbindung mit der Innenseite des distalen Endes des Außenrohrs schafft.

Diese Maßnahme hat den Vorteil, daß ggf. der Verbund aus Fenster und dieses umgebenden Element vorab hergestellt wird, anschließend oder gleichzeitig die äußere umfängliche Schicht auf die Mantelfläche des Elements aufgebracht wird, so daß dieser Verbund dann lediglich noch in das distale Ende des Außenrohrs eingeschoben und, je nach Ausbildung der Schicht, mit diesem verbunden, bspw. verlötet werden muß.

In einer weiteren Ausgestaltung der Erfindung ist die äußere Mantelfläche des Elements mit demselben Material beschichtet, das zur Verbindung zwischen Element und Fenster dient.

Diese Maßnahme hat den Vorteil, daß die dichte Verbindung zwischen Fenster und diesem umgebenden Element einerseits und diesem Verbund mit der Innenseite des Außenrohrs andererseits in einem Arbeitsvorgang durchgeführt werden kann.

In einer weiteren Ausgestaltung der Erfindung ist die lichtundurchlässige Schicht zwischen Fenster und diese umgebenden Element, auf seiten des Elements, als lichtreflektierende Schicht ausgebildet.

Diese Maßnahme hat den erheblichen Vorteil, daß von der Mantelfläche des Fensters kein Beleuchtungslicht absorbiert wird, sondern dieses reflektiert und der Beleuchtung weiterhin zur Verfügung steht.

In einer weiteren Ausgestaltung der Erfindung ist die radial äußere Mantelfläche des Elements mit einer lichtreflektierenden Schicht versehen.

Diese Maßnahme hat denselben vorteilhaften Effekt wie die zuvor genannte Ausgestaltung, in dieser Ausgestaltung wird nunmehr auch von dieser Seite auf die Mantelfläche auftreffendes Beleuchtungslicht reflektiert und der Beleuchtung zur Verfügung gestellt.

Dies ist insbesondere dann von Vorteil, wenn die beiden Mantelflächen nicht parallel zueinander verlaufen, sondern bspw. bei Endoskopen mit abgewinkelten Blickrichtungen, ggf. geneigt zueinander verlaufen.

In einer weiteren Ausgestaltung der Erfindung bestehen die lichtundurchlässige Schicht, und/oder das Material zur Verbindung der Bauelemente des Abschlußelements untereinander und/oder mit dem Außenrohr und/oder die lichtreflektierende Schicht aus einem lötfähigen Material.

Diese Maßnahme hat den Vorteil, daß mit einem lötfähigen Material durch eine Wärmebehandlung und Zuführen von Lot die Verbindung der einzelnen Bauelemente untereinander, die lichtundurchlässige Schicht und, wenn ein geeignetes metallisches lötfähiges Material vorhanden ist, auch zugleich die lichtreflektierende Schicht ausgebildet werden kann. Diese Maßnahme fördert somit die einfache Herstellung der dichtenden Verbindung zwischen den einzelnen Bauelementen des Abschlußelements untereinander und der Verbindung zwischen Abschlußelement und Außenrohr.

In einer weiteren Ausgestaltung der Erfindung ist das Element als Ring ausgebildet, in dem mittig das Fenster passend sitzt.

Diese Maßnahme hat den Vorteil, daß sowohl Ring als auch Fenster einfache Geometrien mit jeweils zylindrischen Mantelflächen aufweisen, die einfach mit den entsprechenden Materialien, sei es eine lichtundurchlässige Schicht oder seien es Materialien zum Verbinden untereinander bzw. zum Verbinden mit dem Außenrohr, zu beschichten sind.

In einer weiteren Ausgestaltung der Erfindung ist bei abgewinkelten Blickrichtungen das Abschlußelement schräg zur Längsachse des Außenrohrs angeordnet.

Diese Maßnahme hat den Vorteil, daß das erfindungsgemäße Abschlußelement auch bei Endoskopen eingesetzt werden kann, die abgewinkelte Blickrichtungen erlauben.

In einer weiteren Ausgestaltung ist das Außenrohr als vom Endoskop abziehbares rohrförmiges Element ausgebildet, das über ein Schaftrohr schiebbar ist, welches das Innenrohr aufweist, und daß das Abschlußelement das abziehbare rohrförmige Element distalseitig hermetisch dicht abschließt.

Diese Maßnahme hat nun den erheblichen Vorteil, daß das Außenrohr, das über das Abschlußelement hermetisch dicht abgeschlossen ist, nach einem Einsatz des Endoskops abgezogen werden kann.

Da nur die Außenseite und der distale Endbereich des Außenrohrs bei einem endoskopischen Eingriff mit den Kontaminationen in Eingriff kommen kann, sind diese auf der Außenseite des Außenrohrs und am Abschlußelement vorhanden. Da nun diese Bauelemente als abziehbares rohrförmiges Element ausgebildet sind, brauchen nur diese Bauelemente sterilisiert werden, wohingegen die verbleibenden Bauteile, also Endoskopkopf samt dem Schaftrohr allenfalls gereinigt werden müssen.

Dadurch ist es nunmehr möglich, im Innenrohr hochtemperaturempfindliche Bauelemente, bspw. entsprechende Chips einzubauen, die dann nicht den hohen Temperaturen bei Autoklaviervorgängen, insbesondere beim Blitzautoklavieren, ausgesetzt sind.

Dies vereinfacht erheblich die Handhabung, insbesondere von Endoskopen, an denen Videokameras angebracht sind, es muß nämlich nicht das gesamte Endoskop zerlegt werden, sondern lediglich das Außenrohr samt dem dieses am distalen Ende hermetisch abschließenden Abschlußelement muß abgezogen werden, um dieses Reinigungs- und Autoklaviervorgängen zu unterwerfen.

In einer weiteren Ausgestaltung der Erfindung ist das rohrförmige Element als steifes Rohr ausgebildet.

Diese Maßnahme hat den Vorteil, daß das abziehbare Außenrohr als steifes stabiles Element ausgebildet ist, somit zum Reinigen und Stabilisieren entsprechend gehandhabt werden kann.

In einer weiteren Ausgestaltung der Erfindung ist das rohrförmige Element als biegeschlaffer Schlauch ausgebildet.

Diese Maßnahme hat den Vorteil, daß das abziehbare Außenrohr als eine Art Überzieher auf das Endoskop aufschiebbar bzw. von diesem abnehmbar ist und bspw. als Wegwerfteil ausgebildet werden kann, so daß Reinigungs- und Sterilisiervorgänge entfallen können.

In einer weiteren Ausgestaltung der Erfindung sind im abziehbaren rohrförmigen Element die Lichtleiter montiert.

Diese Maßnahme hat den Vorteil, daß eine Baugruppe geschaffen wird, die auf ein Endoskop, das lediglich die optischen Bauelemente enthält, als kompakte Einheit aufgeschoben werden kann.

In einer weiteren Ausgestaltung der Erfindung sind am Abschlußelement Anlagen vorgesehen, an die das distale Ende des Schaftrohrs definiert positioniert zur Anlage kommt.

Diese Maßnahme hat den Vorteil, daß bei der Ausgestaltung mit dem abziehbaren rohrförmigen Element das Außenrohr in einer genau definierten Position relativ zum Schaftrohr kommt, in dessen Innenrohr das optische System aufgenommen ist. Das bedeutet, daß das Fenster des Abschlußelements in einer exakt definierten Position vor dem Innenrohr des Schaftrohrs zum Liegen kommt, somit keine Beeinflussung des Bildes erfolgt. In diesem Fall ist dann das Innenrohr über eine Abschlußscheibe verschlossen.

In einer weiteren Ausgestaltung der Erfindung ist ein Lichtanschluß im proximalen Bereich des Endoskops, zu dem das proximale Ende der Lichtleiter geführt ist, durch ein Lichtanschlußfenster ebenfalls hermetisch dicht abgeschlossen.

Diese Maßnahme hat den Vorteil, daß der gegenüber Eindringen von Kontaminationen kritische Raum, in dem die Lichtleiter aufgenommen sind, nun an beiden Enden hermetisch dicht abgeschlossen ist, also sowohl am proximalen als auch am distalen Ende.

In einer weiteren Ausgestaltung ist das Lichtanschlußfenster des Lichtanschlusses gleichermaßen wie das Abschlußelement hermetisch dichtend angebracht.

Diese Maßnahme hat nun den Vorteil, daß beide dichten Verbindungen, also sowohl die des distalseitigen Abschlußelements als auch die des proximalen Abschlusses des Lichtanschlusses gleichzeitig herbeigeführt werden können, bspw. durch einen Wärmebehandlungsvorgang zum Verlöten der Fenster mit den entsprechenden Bauelementen.

In einer weiteren Ausgestaltung der Erfindung ist das Abschlußelement in dem Bereich, durch den das Beleuchtungslicht hindurchgeführt wird, als optisch wirksames Element, insbesondere als Diffusor oder Fokussierlinse ausgebildet.

Diese Maßnahme hat den Vorteil, daß das Abschlußelement im Beleuchtungslichtdurchtrittsbereich nicht nur als Lichtdurchlaß dient, sondern als optisch aktives Element ausgebildet ist, das dem Beleuchtungslicht gewisse Eigenschaften verleiht.

In einer weiteren Ausgestaltung der Erfindung ist das Abschlußelement in dem Bereich, durch den das Beleuchtungslicht hindurchgeführt wird, derart geformt, daß eine Lenkung des Beleuchtungslichts aus der Lichtachse heraus erfolgt.

Diese Maßnahme hat den Vorteil, daß allein schon durch die Formgebung dem Beleuchtungslicht gewisse Richtungseigenschaften verliehen werden, die selbstverständlich auch mit der zuvor genannten Maßnahme noch zusätzlich kombiniert werden können.

In einer weiteren Ausgestaltung der Erfindung ist die Ausformung derart, daß an der Außenseite und/oder an der Innenseite des Abschlußelements Abflachungen vorhanden sind.

Diese Maßnahme hat den Vorteil, daß derartige Abflachungen sehr einfach zu bewerkstelligen sind und daß dem Beleuchtungslicht durch die Geometrie der Abflachungen bzw. des Anstellwinkels der Abflachungen relativ zur Lichtleiterachse eine gewünschte Richtungskomponente auferlegt wird.

In einer weiteren Ausgestaltung der Erfindung ist das Fenster als optisch wirksames Element, insbesondere als Linse und/oder als Filter ausgebildet.

Diese Maßnahme hat den Vorteil, daß das Fenster nicht nur für den Bildeintritt dient, sondern schon bereits einen Teil der optischen Bauelemente darstellt, so daß insgesamt gesehen die Gesamtzahl der optischen Bauelemente reduziert werden kann.

In einer weiteren Ausgestaltung der Erfindung ist das Abschlußelement IR-Licht absorbierend ausgebildet.

Diese Maßnahme hat nun den erheblichen Vorteil, daß aufgrund dieser IR-Lichtabsorption, also von Wärmestrahlung, das Abschlußelement erwärmt wird.

Bei Endoskopen besteht die Gefahr, daß das distale Abschlußelement beim Einführen in einen Körper, dessen Temperatur üblicherweise höher ist als die Umgebungstemperatur, in der das Endoskop sich vorher befunden hat, beschlägt. Dies kann nun dadurch ausgeschlossen werden, daß durch Absorption von Wärme aus dem durch das Abschlußelement hindurchtretenden Licht es auf eine solche Temperatur erwärmt wird, daß kein Beschlagen stattfindet. Somit dient das erfindungsgemäße Abschlußelement zusätzlich als "Anti-Fog-Einheit".

Wie zuvor erwähnt, steht ein relativ großer Raum um das mittige Fenster herum als Durchtrittsfläche für das Beleuchtungslicht zur Verfügung. Durch gezielte spektrale Zusammensetzung des Beleuchtungslichts kann nun ein für den jeweiligen Anwendungsfall geeigneter IR-Lichtanteil im Beleuchtungslicht vorhanden sein, der vom Abschlußelement absorbiert wird und durch Wärmeleitung zur Erwärmung des Abschlußfensters, also des Abschlußelements über dessen gesamten Querschnitt, herangezogen wird. Dadurch können dann auch örtliche Überhitzungen oder dgl. ausgeschlossen werden.

In einer weiteren Ausgestaltung ist die lichtundurchlässige Schicht, die das Fenster umgibt, IR-Licht durchlässig.

Diese Maßnahme hat nun den Vorteil, daß die an sich lichtundurchlässige Schicht den Übertritt von IR-Strahlung aus dem Bereich des Abschlußelement, durch den das Beleuchtungslicht hindurchtritt, in Richtung des Fensters fördert und somit für eine gezielte und rasche Erwärmung dieses besonders durch Feuchtigkeitsbeschlag beeinträchtigten Bereiches sorgt.

In einer weiteren Ausgestaltung der Erfindung ist die Innenseite des Fensters und/oder die Außenseite des Abschlußelements mit einer IR-Licht reflektierenden Schicht beschichtet.

Diese Maßnahme hat nun den erheblichen Vorteil, daß in das Abschlußelement eingekoppeltes IR-Licht nicht wieder von diesem austreten kann, sondern durch die IR-reflektierenden Schichten mehrfach im Fenster reflektiert wird und für dessen rasche und gezielte Erwärmung sorgt. Insbesondere werden IR-Lichtanteile des Beleuchtungslichtes an der Austrittsseite des das Fenster umgebenden Bereichs reflektiert und somit im Abschlußelement gefangen.

Das Abschlußfenster arbeitet als eine Art IR-Lichtfalle, das aus dem Beleuchtungslicht IR-Licht entnimmt, gezielt dem mittigen Fenster, das das Bildleitsystem abschließt, zuführt und dort durch Reflexion für eine Erwärmung sorgt.

In einer weiteren Ausgestaltung der Erfindung ist die Innenseite des Abschlußelements in dem Bereich, in dem die Lichtleiter münden, mit einer semipermeablen Schicht beschichtet, die in Beleuchtungsrichtung lichtdurchlässig ist und in Gegenrichtung sperrt.

Diese Maßnahme fördert die zuvor beschriebene Wirkung des Abschlußelementes als "IR-Falle".

Das Abschlußelement ist also insgesamt so ausgebildet, daß das Beleuchtungslicht in Beleuchtungsrichtung hindurchtreten kann, das IR-Licht absorbiert wird und zur Erwärmung herangezogen wird und ein Austritt von absorbiertem IR-Licht über die gesamten Außenseiten gehindert ist, also über die gesamte Innenseite und über die gesamte Außenseite des Abschlußelements ein Austritt aufgrund der reflektierenden Ausbildung der Außenseiten gehindert ist.

In einer besonderen Ausgestaltung der Erfindung wird ein Verfahren zur Herstellung eines Abschlußelements zum Verschließen des distalen Endes eines Endoskops vorgeschlagen, das gekennzeichnet ist durch
a) Bereitstellen eines langen stabförmigen lichtdurchlässigen Materials mit dem Querschnitt des Fensters,
b) Bereitstellen eines langen rohrförmigen lichtdurchlässigen Materials, in dessen inneren Hohlraum das lange stabförmige Material etwa passend einführbar ist,
c) Vorsehen einer lichtundurchlässigen Schicht zwischen Außenseite des stabförmigen Materials und Innenseite des Hohlraumes,
d) Ineinanderschieben von stabförmigem Material und rohrförmigem Material,
e) festes und dichtes Verbinden der Außenseite des stabförmigen Materials mit der Innenseite des Hohlraumes, und
f) Auftrennen des Zusammenbaus von stabförmigem Material und rohrförmigem Material in einzelne scheibenförmige Abschlußelemente.

Diese Maßnahme hat den Vorteil, daß auf einfache Weise die einzelnen Abschlußelemente hergestellt werden können, was zur Lösung der Aufgabe bezüglich des einfach zu bewerkstelligenden Abschlusses beiträgt. Stabförmige und rohrförmige lichtdurchlässige Materialien sind großtechnisch einfach und maßgenau und in entsprechend großer Länge herzustellen, so daß aus einem einzigen Zusammenbau sehr viele Abschlußelemente erhalten werden können.

Nach Bewerkstelligung des Zusammenbaus kann überprüft werden, ob die Verbindung sowohl dicht als auch lichtundurchlässig ist und dann kann die Auftrennung in die einzelnen Abschlußelemente durchgeführt werden.

In einer besonderen Ausgestaltung des Verfahrens wird in Schritt c) die Außenseite des stabförmigen Materials mit der lichtundurchlässigen Schicht versehen.

Diese Maßnahme hat den Vorteil, daß dies sehr einfach durchzuführen ist, bspw. indem der Stab in eine entsprechende Lösung eingetaucht wird und dann das entsprechende Material gleichmäßig und in vorbestimmter Dicke auf der Außenseite des stabförmigen Materials aufgebracht ist. Schon zu diesem Zeitpunkt kann eine entsprechende Kontrolle durchgeführt werden.

Nach dem Verbinden, sei es durch einen Verklebevorgang, sei es durch einen Sintervorgang, ist die Verbindungsstelle visuell bzw. optisch einwandfrei zu kontrollieren, was ebenfalls der einfachen Herstellung zuträglich ist.

Es versteht sich, daß die vorstehend genannten und die nachstehend noch zu erläuternden Merkmale nicht nur in der angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: stark schematisiert einen Längsschnitt eines Endoskops mit einem erfindungsgemäßen Abschlußelement,
- Fig. 2: einen stark vergrößerten Längsschnitt des Endoskops von Fig. 1 im Bereich dessen distalem Ende,
- Fig. 3: eine der Schnittdarstellung von Fig. 2 vergleichbare Darstellung der Bauelemente des Abschlußelements in Explosionsdarstellung,
- Fig. 4: einen Längsschnitt eines distalen Endbereichs eines weiteren Ausführungsbeispiels eines erfindungsgemäßen Endoskops mit abziehbarem Außenrohr, dessen Ende durch ein erfindungsgemäßes Abschlußelement hermetisch dicht verschlossen ist,
- Fig. 5: einen der Darstellung von Fig. 2 vergleichbaren Längsschnitt eines weiteren Ausführungsbeispiels eines Endoskops mit abgewinkelter Blickrichtung,
- Fig. 6: einen der Darstellung von Fig. 5 vergleichbaren Schnitt eines weiteren Ausführungsbeispiels eines Endoskops mit abgewinkelter Blickrichtung,
- Fig. 7: eine mit den Schnittdarstellungen von Fig. 5 und 6 vergleichbare Darstellung eines weiteren Ausführungsbeispiels, eines Endoskops mit abgewinkelter Blickrichtung,
- Fig. 8: eine der Schnittdarstellung von Fig. 2 vergleichbare Darstellung eines weiteren Ausführungsbeispiels, mit einem optisch wirksamen Fenster,
- Fig. 9: einen der Schnittdarstellung von Fig. 8 vergleichbaren Schnitt eines weiteren Ausführungsbeispiels, mit Abflachungen im Bereich des Abschlußelements, in dem das Beleuchtungslicht austritt,
- Fig. 10: eine der Darstellung von Fig. 4 vergleichbare Darstellung eines Ausführungsbeispiels, mit abziehbarem Außenrohr, in dem die Lichtleiter aufgenommen sind, und
- Fig. 11: einen Querschnitt eines distalen Endes eines weiteren Ausführungsbeispiels eines Endoskops durch das Abschlußelement.

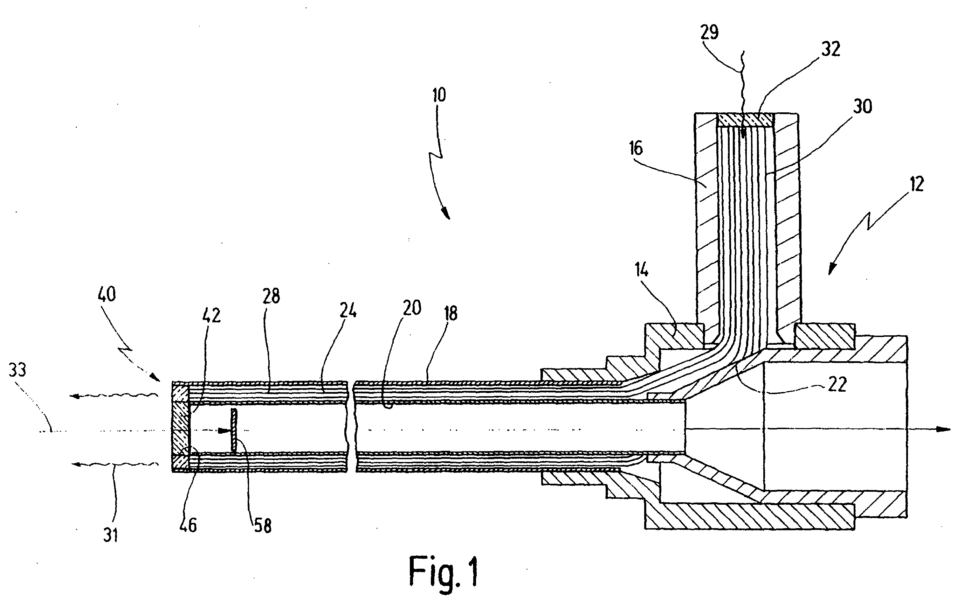

Ein in den Fig. 1 bis 3 dargestelltes Endoskop 10 weist proximal einen Kopf 12 auf.

Der Kopf 12 weist ein Gehäuse 14 auf, von dem seitlich ein Lichtanschluß 16 in Form eines Rohrstutzens vorspringt.

Das Gehäuse 14 ist mit einem schaftartigen Außenrohr 18 fest und zur Außenseite hin dicht verbunden, wozu dieses in das Gehäuse 14 eingeschoben und mit diese verlötet ist.

Im Außenrohr 18 ist koaxial zu diesem ein durchmessergeringeres Innenrohr 20 aufgenommen, das mit einem Okularansatz 22 fest verbunden ist, der im Innenraum des Gehäuses 14 aufgenommen ist. Proximalseitig ist der Okularansatz 22 mit einem Okular bzw. einer daran angesetzten Okularmuschel dicht verbunden, wenn das Innenrohr 20 zur Aufnahme eines hier nicht näher dargestellten optischen Linsensystems dient.

Aufgrund der Tatsache, daß der Außendurchmesser des Innenrohrs 20 geringer ist als der lichte Innendurchmesser des Außenrohrs 18, entsteht zwischen diesen ein Zwischenraum 24.

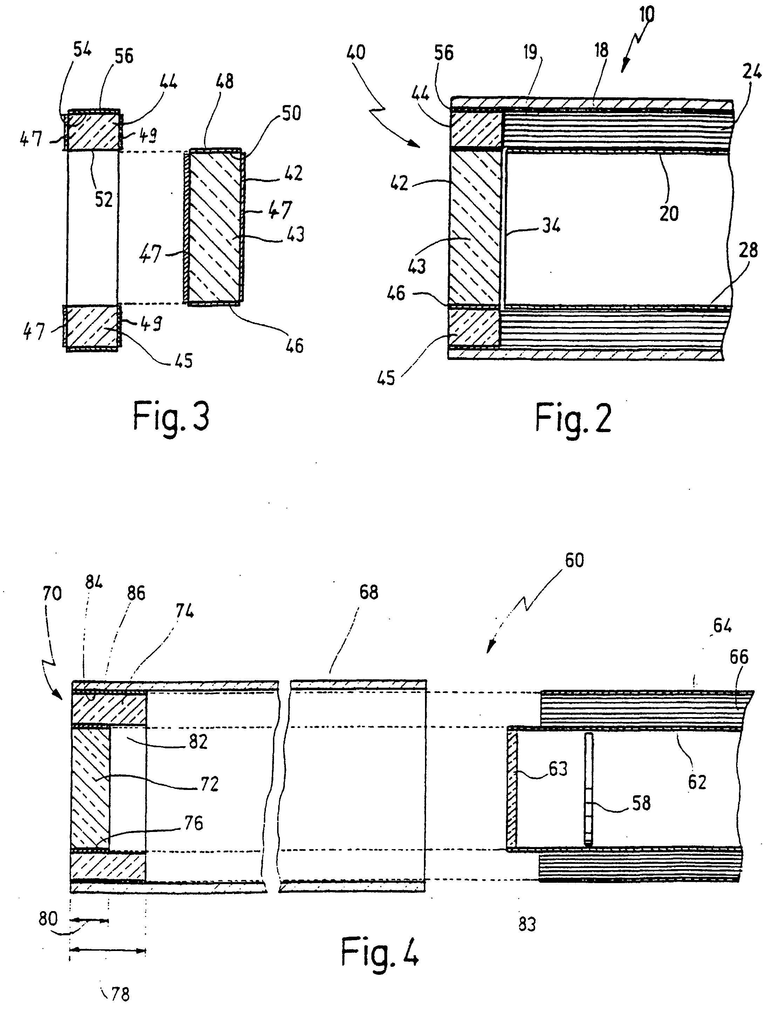

Der Zwischenraum 24 dient dazu, Lichtleiter 28 in Form eines Bündels an Glasfasern 30 aufzunehmen, die vom äußeren Ende des Lichtanschlusses 16 bis zum äußeren proximalen Ende des Innenrohrs 20 geführt sind, wie das insbesondere in der Schnittdarstellung von Fig. 2 ersichtlich ist. Ein Lichtanschlußfenster 32 verschließt proximalseitig hermetisch und dicht den Lichtanschluß 16, erlaubt jedoch einen Eintritt von Licht ausgehend von einer Lichtquelle in die Glasfasern, wie das in Fig. 1 durch einen Pfeil 29 angedeutet ist.

Distalseitig ist das Außenrohr 18 über ein Abschlußelement 40 hermetisch dicht abgeschlossen.

Dazu überragt das Außenrohr 18 das Innenrohr 20 distalseitig, und zwar um die axiale Dicke des Abschlußelements 40.

Das Abschlußelement 40 besteht aus einem Fenster 42 aus Glas, das im dargestellten Ausführungsbeispiel die Form einer kreisrunden ebenen Scheibe 43 aufweist.

Das die Scheibe 43 umgebende Element 44 ist als Ring 45 ausgebildet, der ebenfalls aus Glas hergestellt ist.

Der Außendurchmesser der Scheibe 43 entspricht etwa dem lichten Innendurchmesser des Innenrohrs 20.

Der Außendurchmesser des Ringes 45 entspricht etwa dem lichten Innendurchmesser des Außenrohrs 18.

Die äußere Mantelfläche 50 der Scheibe 43 ist mit einer lichtundurchlässigen Schicht 46 versehen. Die lichtundurchlässige Schicht 46 besteht aus einem Rohrabschnitt 48 eines metallischen Materials mit Löteigenschaften, das mit der Scheibe 43 verschmolzen ist. Gleichermaßen ist die äußere Mantelfläche 54 des Ringes 45 mit einer metallischen Schicht 56 versehen.

Aus der Darstellung von Fig. 3 ist ersichtlich, daß die Scheibe 43 samt deren darauf aufgebrachten lichtundurchlässigen Schicht 46 passend in den Ring 45 eingeschoben werden kann.

Der Zusammenbau aus Scheibe 43 und Ring 45 samt der äußeren umfänglichen Schicht 56 kann gerade in den über das Innenrohr 20 überstehenden Abschnitt in das Außenrohr 18 eingeschoben werden. Nach einer Wärmebehandlung ist die Mantelfläche 50 der Scheibe 43 hermetisch dicht mit dem Ring 45 verbunden, dieser wiederum hermetisch dicht mit der Innenseite des Außenrohrs 18, so daß dadurch ein hermetisch dichter Abschluß erfolgt.

Die lichtundurchlässige Schicht 46 stellt, wie das insbesondere aus Fig. 2 ersichtlich ist, eine Art Verlängerung des Innenrohrs 20 dar, schirmt somit den Innenraum des Innenrohrs 20 vor radialem Eintritt von Beleuchtungslicht ab, das durch den Ring 45 austritt, wie das in Fig. 1 durch Pfeile 31 angedeutet ist. Durch die Scheibe 43 kann das Bild in das Innere des Innenrohrs 20 eintreten, wie das in Fig. 1 durch einen Pfeil 33 angedeutet ist, und dann über entsprechende optische Bauelemente, wobei nur ein Bauelement 58 schematisch angedeutet ist, zum proximalen Ende des Kopfes 12 geführt werden.

Wie das aus der Darstellung von Fig. 2 ersichtlich ist, steht das distale Ende 34 des Innenrohrs 20 weder mit dem Abschlußelement 40 noch mit dem Außenrohr 18 in fester Verbindung, so daß keine Spannungen in diesem Bereich aufgrund unterschiedlicher Längenausdehnungen bei Temperaturschwankungen auftreten können. Die Breite des Spaltes ist Gründen der Darstellung in Fig. 2 groß gehalten.

Da das distale Ende des Zwischenraumes 24, an dem die Glasfasern 30 enden, hermetisch gegen die Außenseite abgeschirmt ist, muß die Verbindung bzw. Lagefixierung der Glasfasern 30 nicht den hohen Dichtigkeitsanforderungen wie beim eingangs erwähnten Stand der Technik standhalten.

In Fig. 3 ist dargestellt, wie das Abschlußelement 40 zusätzlich als "Anti-Fog-Einheit" ausgebildet werden kann. Sowohl die Innen- als auch die Außenseite des Fensters 42 ist mit einer Schicht 47 aus IR-Licht reflektierendem Material beschichtet. Auch die Außenseite des Ringes 45 ist mit einer solchen Schicht 47 beschichtet.

Die lichtundurchlässige Schicht 46 ist so ausgebildet, daß diese IR-Licht durchlässig ist, sichtbares Licht jedoch sperrt.

Ferner ist die Innenseite des Ringes 45 mit einer semipermeablen Schicht 49 beschichtet ist. Diese Schicht 49 läßt das gesamte Beleuchtungslicht der Lichtleiter 28 in Beleuchtungsrichtung durchtreten, sperrt aber in Gegenrichtung.

Wird nun der Zusammenbau aus Ring 45 und Scheibe 43 in das Endoskop 10 eingesetzt, wie das in Fig. 2 dargestellt ist (die zusätzlichen Schichten sind der Übersichtlichkeit halber hier nicht dargestellt), so ersichtlich, daß das Abschlußelement 40 als eine IR-Falle dient. Das von den Lichtleitern 28 herangeführte Beleuchtungslicht tritt, im Bereich des Ringes 45, durch das Abschlußelement 40 hindurch. IR-Lichtanteile des Beleuchtungslichtes werden von der Schicht 47 reflektiert und führen zu einer Erwärmung des Ringes 45. Die semipermeable Schicht 49 reflektiert wieder dieses reflektierte Licht. Dadurch, daß die lichtundurchlässige Schicht 46 IR-Licht durchlässig ist, findet auch eine Reflexion und somit Absorption von IR-Licht in der Scheibe 43 statt. Da die Scheibe 43 beidseitig mit IR-Licht reflektierender Schicht 47 beschichtet ist, wird das über das Beleuchtungslicht eingekoppelte IR-Licht mehrfach reflektiert und führt zu einer raschen und gleichmäßigen Erwärmung des Zusammenbaus aus Scheibe 43 und Ring 45, also des gesamten Abschlußelementes 40, so daß ein Beschlagen der Außenseite verhindert ist.

Das Abschlußelement 40 kann dadurch hergestellt werden, daß zunächst ein langes stabförmiges lichtdurchlässiges Glasmaterial mit dem Querschnitt der Scheibe 43 bereitgestellt wird und dessen Außenseite mit der lichtundurchlässigen Schicht 46 beschichtet wird. Dieser Stab wird dann in ein Rohr eingeschoben, das der Kontur des Ringes 45 entspricht. Dieser Zusammenbau wird dann zur Bewerkstelligung der dichten und festen Verbindung zwischen der Außenseite der Scheibe 43 und der Innenseite des Ringes 45 entsprechend behandelt, je nachdem wie die lichtundurchlässige Schicht 46 ausgebildet ist, also als Klebeverbindung, als Löt- oder Sinterverbindung. Anschließend wird dieser Zusammenbau in scheibenförmige Abschlußelemente 40 aufgetrennt und dann können die entsprechenden Beschichtungen aufgebracht werden, falls die Ausgestaltung des Abschlußelementes als IR-Falle bzw. "Anti-Fog-Einheit" gewünscht ist.

In Fig. 4 ist ein weiteres Ausführungsbeispiel eines Endoskops 60 dargestellt.

Das Endoskop 60 weist ein Innenrohr 62 auf, das distalseitig über ein Abschlußfenster 63 dicht abgeschlossen ist. Das Innenrohr 62 ist von einem äußeren Schaftrohr 64 umgeben, in dem Zwischenraum zwischen äußerem Schaftrohr 64 und Innenrohr 62 sind die Lichtleiter 66 aufgenommen.

Wie aus Fig. 4 ersichtlich ist, überragt das Innenrohr 62 das distale Ende der Lichtleiter 66 um ein bestimmtes Maß.

Über das äußere Schaftrohr 64 ist ein von diesem abziehbares rohrförmiges Element 68 schiebbar, dessen Länge der Gesamtlänge des Schaftes des Endoskops 60 entspricht.

Das an der proximalen Seite offene rohrförmige Element 68 ist distalseitig über ein Abschlußelement 70 hermetisch dicht zur Außenseite hin abgeschlossen.

Das Abschlußelement 70 weist, wie zuvor beschrieben, ein Fenster 72 in Form einer Scheibe auf, das in einem Ringelement 74 aufgenommen ist. Das Fenster 72 ist, ebenfalls wie zuvor beschrieben, von einer lichtundurchlässigen Schicht 76 umgeben.

Die axiale Länge 78 des Ringelements 74 ist dabei größer als die axiale Länge 80 des Fensters 72, und zwar um das Maß, um das das Innenrohr 62 über die Lichtleiter 66 hinaussteht.

Dadurch ist eine Anlage 82 geschaffen, in die der Vorsprung 83 definiert zum Liegen kommen kann.

Da das Abschlußelement 70 über die äußere umfängliche Schicht 86 an der äußeren Mantelfläche des Ringelements 74 hermetisch dicht mit dem rohrförmigen Element 68 verbunden ist und das Fenster 72 über die lichtundurchlässige Schicht 76 ebenfalls hermetisch dichtend mit dem Ringelement 74 verbunden ist, entsteht ein hermetisch dichter distaler Abschluß des abziehbaren rohrförmigen Elements 68. Ist das Element 68 vollständig auf das äußere Schaftrohr 64 aufgeschoben, kommt das Fenster 72 unmittelbar am Abschlußfenster 63 des Innenrohrs 62 zum Liegen. Die lichtundurchlässige Schicht 76 bildet dann wieder eine Verlängerung des Innenrohrs 62 und übernimmt somit wieder die Abschirmfunktion gegenüber dem durch das Ringelement 74 austretenden Beleuchtungslicht. Wurde mit dem Endoskop 60 ein Eingriff durchgeführt, kann anschließend das abziehbare Element 68 samt Abschlußelement 70 abgezogen und gereinigt bzw. sterilisiert werden. Da das Element 68 den gesamten Schaft des Endoskops 60 bedeckt, ist der Kopf des Endoskops 60 bzw. dessen äußeres Schaftrohr 64 nicht verschmutzt oder durch Bakterien kontaminiert, so daß eine einfache Reinigung ausreichend ist. Daher kann im Innenrohr 62 bspw. ein temperaturempfindliches optisches Element 58' in Form eines CCD-Sensors eingebaut werden, da dieses Bauteil des Endoskops 60 nicht den hohen Autoklaviertemperaturen unterworfen werden muß.

Im dargestellten Ausführungsbeispiel ist das Element 68 als Metallrohr ausgebildet. Es kann auch aus einem biegeschlaffen Kunststoffmaterial bestehen, das auf das Schaftrohr 64 als Überzieher aufbringbar ist und nach Abziehen verworfen wird.

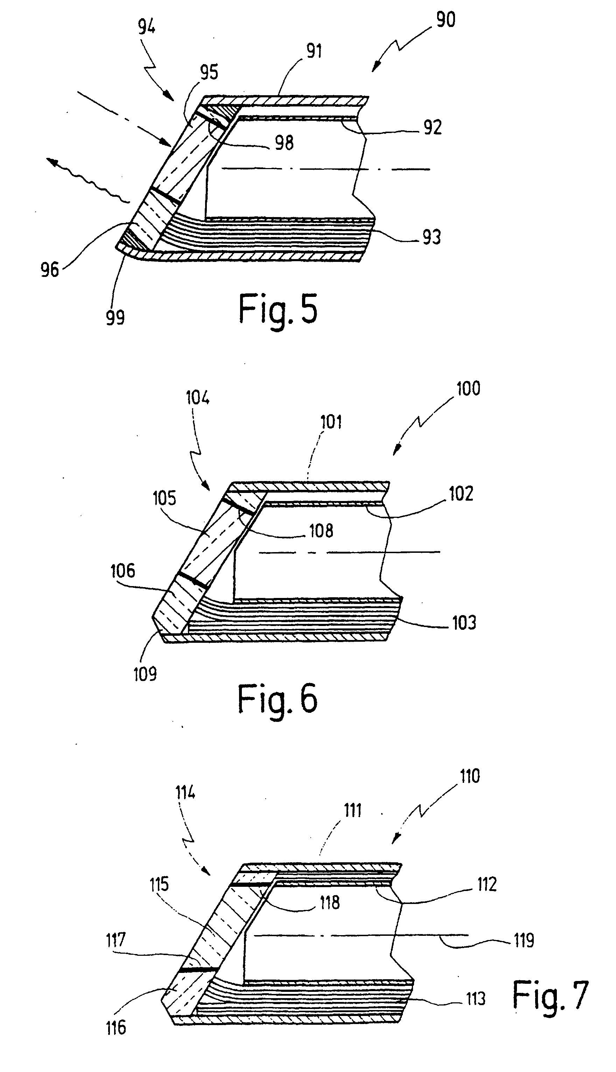

In Fig. 5 ist ein distaler Endbereich eines Endoskops 90 dargestellt, das eine abgewinkelte Blickrichtung erlaubt.

Im Außenrohr 91 ist ein Innenrohr 92 aufgenommen, wobei im Zwischenraum Lichtleiter 93 angeordnet sind.

Ein Abschlußelement 94 schließt das distale Ende des Außenrohrs 91 hermetisch dicht ab, wie das auch zuvor beschrieben wurde, d.h. das Abschlußelement 94 weist eine Scheibe 95 und einen diesen umrundenden Ring 96 auf. Eine lichtundurchlässige Schicht 98 sorgt für die Abschirmung gegenüber dem Beleuchtungslicht.

Ein gebogenes Ende 99 des Außenrohrs 91 umgreift die äußere Mantelfläche des Abschlußelements 94.

Bei einem in Fig. 6 dargestellten weiteren Ausführungsbeispiel eines Endoskops 100 ist ebenfalls eine abgewinkelte Blickrichtung möglich, das Außenrohr 101, das ein Innenrohr 102 und entsprechende Lichtleiter 103 aufnimmt, ist hermetisch dicht über ein Abschlußelement 104 abgeschlossen, das aus einer mittigen Scheibe 105 besteht, die von einem Ring 106 umgeben wird.

Eine lichtundurchlässige Schicht 108 sorgt für die Abschirmung des Beleuchtungslichts.

Eine Bördelung 109 des Außenrohrs 101 umgreift das Abschlußelement 104.

Bei den in Fig. 5 und 6 gezeigten Ausführungsbeispielen ist die lichtundurchlässige Schicht 98 bzw. 108 bezüglich ihrer Axialerstreckung in Richtung der Abwicklung ausgerichtet.

Bei dem in Fig. 7 dargestellten Ausführungsbeispiel eines Endoskops 110 ist in einem Außenrohr 111, das ein Innenrohr 112 und Lichtleiter 113 aufnimmt, ein Abschlußelement 114 vorgesehen, das aus einer Scheibe 115 und einem Ring 116 ausgebildet ist. Eine Mantelfläche 117 der lichtundurchlässigen Schicht 118 ist, obwohl dieses Endoskop 110 eine abgewinkelte Blickrichtung erlaubt, in Richtung der Längsachse 119 des Außenrohrs 111 ausgerichtet. Dadurch wird eine besonders gute Abschirmung gegenüber dem Beleuchtungslicht ermöglicht.

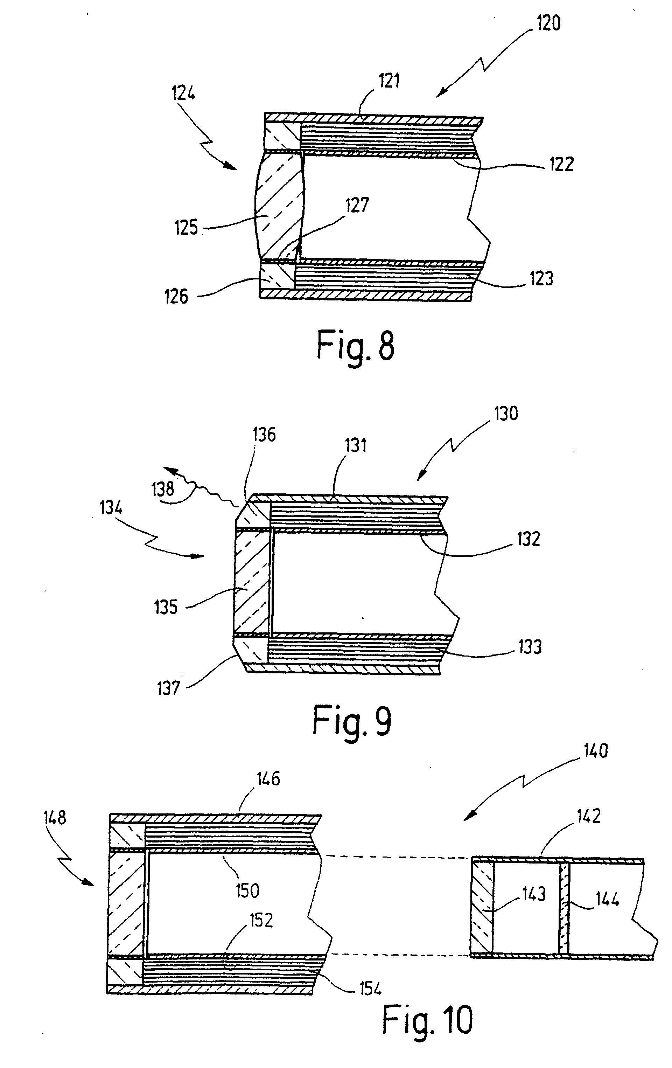

Bei dem in Fig. 8 dargestellten Ausführungsbeispiel eines Endoskops 120 ist in einem Außenrohr 121, das ein Innenrohr 122 und Lichtleiter 123 aufnimmt, ein Abschlußelement 124 vorgesehen, dessen Fenster 125 als Linse ausgebildet ist. Das Fenster 125 ist von einem Ring 126 umgeben, zwischen diesen liegt dann wieder die lichtundurchlässige Schicht 127. Das Fenster 125 ist damit schon Teil des optischen Systems im Innenrohr 122.

Bei dem in Fig. 9 dargestellten Ausführungsbeispiel eines Endoskops 130 ist in einem Außenrohr 131, das ein Innenrohr 132 und Lichtleiter 133 aufnimmt, ein Abschlußelement 134 vorgesehen, dessen Fenster 135 als planebene Scheibe ausgebildet ist. Der das Fenster 135 umgebende Ring 136 weist auf der Außenseite eine Abflachung 137 auf. Durch die Abflachung 137 ist es möglich, das von den Lichtleitern 133 kommende Licht seitlich abzuleiten, wie das durch einen Pfeil 138 angedeutet ist.

Bei dem in Fig. 10 dargestellten Ausführungsbeispiel eines Endoskops 140 ist dasselbe Prinzip wie bereits in Zusammenhang mit Fig. 4 beschrieben verwirklicht.

Ein Innenrohr 142, das distal über ein Abschlußfenster 143 hermetisch dicht abgeschlossen ist, dient zur Aufnahme optischer Bauelemente 144. Ein Außenrohr in Form eines rohrförmigen Elements 146 ist distalseitig mit einem Abschlußelement 148 abgeschlossen, wie es in Zusammenhang mit den Fig. 1 bis 3 beschrieben ist.

Im rohrförmigen Element 146 ist ein weiteres inneres Rohr 150 eingeschoben, in dem Zwischenraum 152 zwischen Rohr 150 und äußerem rohrförmigem Element 146 sind Lichtleiter 154 aufgenommen.

Dadurch ist eine kompakte Baueinheit geschaffen, die auf ein Innenrohr 142 aufgeschoben werden kann, das ausschließlich optische Elemente, insbesondere elektronische Elemente enthält.

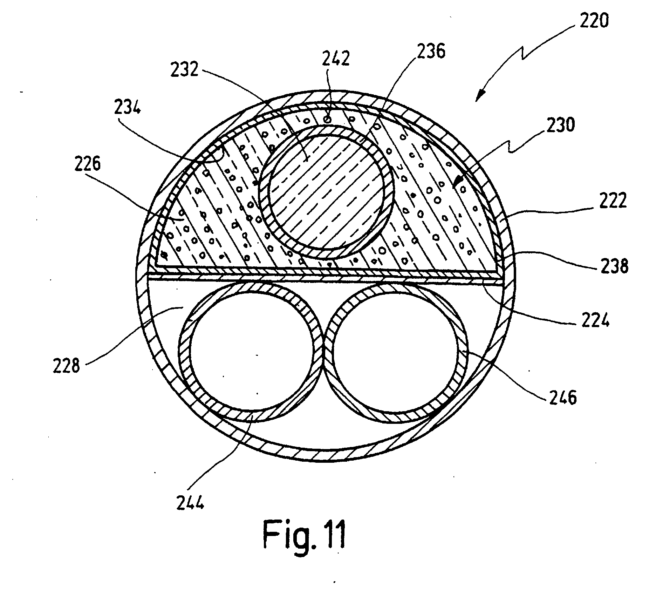

In Fig. 11 ist ein Querschnitt eines distalen Endes eines weiteren Ausführungsbeispiels eines Endoskops 220 dargestellt, das ein Außenrohr 222 aufweist, das mittig über eine Trennwand 224 in zwei voneinander getrennte Bereiche 226 und 228 aufgeteilt ist. Der Bereich 226 ist distalseitig hermetisch dicht durch ein Abschlußelement 230 abgeschlossen.

Das Abschlußelement 230, das die Form einer Halbkreisfläche aufweist, enthält mittig eine Scheibe 232, die vor einem distalen Ende eines hier nicht dargestellten und ersichtlichen Endes eines Innenrohrs zum Liegen kommt, das im Bereich 226 angeordnet ist. Eine lichtundurchlässige Schicht 236 umrundet die Scheibe 232.

Die Scheibe 232 ist hermetisch dichtend in einem diese umgebenden Element 234 aufgenommen, das zusammen mit der Scheibe 232 das Abschlußelement 230 bildet. Das Element 234 ist über eine Schicht 238 mit der Innenseite des Außenrohrs 222 bzw. der dem Bereich 226 zugewandten Seite der Trennwand 224 hermetisch dichtend verbunden.

Im Bereich 226, um das hier nicht dargestellte Innenrohr herum, sind Lichtleiter 242 in Form von Glasfasern angeordnet, die unmittelbar am Abschlußelement 230 enden.

Im Bereich 228 sind Führungsrohre 244, 246 angeordnet, die zur Außenseite hin offen sind, durch die bspw. Instrumente oder gasförmige oder flüssige Medien geführt werden können.

In der Schnittdarstellung von Fig. 11 ist die Scheibe 232 etwa mittig im Element 234 angeordnet, sie kann auch am oberen oder am unteren Ende angeordnet sein, je nachdem wie das Innenrohr verläuft.

## Patentansprüche

1. Endoskop, mit einem Kopf (12) am proximalen Ende, mit einem mit dem Kopf (12) verbundenen Außenrohr (18, 91, 101, 111, 121, 131, 222), mit einem im Außenrohr (18, 91, 101, 111, 121, 131, 222) aufgenommenen Innenrohr (20, 62, 92, 102, 112, 122, 132, 142), in welchem optische Bauelemente (58, 58', 144) aufgenommen sind, wobei am distalen Ende des Innenrohrs (20, 62, 92, 102, 112, 122, 132, 142) ein Fenster (42, 72, 125) aus lichtdurchlässigem Material angeordnet ist, sowie mit Lichtleitern (28, 66, 93, 103, 113, 123, 133, 154, 242), die in einem Zwischenraum (24) zwischen Innenrohr (20, 62, 92, 102, 112, 122, 132, 142) und Außenrohr (18, 91, 101, 111, 121, 131, 222) aufgenommen und axial bis zum distalen Ende des Zwischenraumes (24, 152) geführt sind, wobei ein Abschlußelement (40, 70, 94, 104, 114, 124, 134, 148, 230) aus lichtdurchlässigem Material vorgesehen ist, das das distale Ende des Außenrohrs (18, 91, 101, 111, 121, 131, 222), zumindest im Bereich des Innenrohrs und des die Lichtleiter aufnehmenden Zwischenraums (24, 152), hermetisch dicht abschließt, daß das Abschlußelement (40, 70, 94, 104, 114, 124, 134, 148, 230) im Bereich des distalen Endes des Innenrohrs das Fenster (42, 72, 125) aufweist, über das Licht in das Innenrohr eintreten kann, und daß das Fenster (42, 72, 125) umfänglich von einer lichtundurchlässigen Schicht (46, 76, 98, 108, 118, 127, 236) umgeben ist, die das Fenster (42, 72, 125) radial vor Eintritt von Beleuchtungslicht abschirmt, das, von den Lichtleitern kommend, durch das Abschlußelement hindurchgeführt wird und am distalen Ende austritt, **dadurch gekennzeichnet, daß** das Abschlußelement (40, 70, 94, 104, 114, 124, 134, 148, 230) aus lediglich zwei Bauelementen aufgebaut ist, nämlich aus dem mittigen Fenster (42, 72, 125), das in das Abschlußelement (40, 70, 94, 104, 114, 124, 134, 148, 230) eingesetzt ist und mit diesem nur über die umfängliche lichtundurchlässige Schicht (46, 76, 98, 108, 118, 127, 236) verbunden ist, und daß das Abschlußelement (40, 70, 94, 104, 114, 124, 134, 148, 230) nur mit seiner äußeren Umfangskante mit dem Außenrohr (18, 91, 101, 111, 121, 131, 222) verbunden ist.

2. Endoskop nach Anspruch 1, **dadurch gekennzeichnet, daß** das Abschlußelement (40, 70, 94, 104, 114, 130) aus dem Fenster (42, 72, 125), einer dessen umfängliche Mantelfläche (50) bedeckenden lichtundurchlässigen Schicht (46, 76, 98, 108, 118, 127, 236) und einem dieses umgebenden Element (44, 74) zusammengesetzt ist.

3. Endoskop nach Anspruch 1 oder 2, **dadurch gekennzeichnet, daß** die lichtundurchlässige Schicht derart ausgebildet ist, daß diese, neben der Abschirmung, auch für eine hermetisch dichte Verbindung zwischen Fenster (42, 72) und dieses umgebenden Element (44, 74) sorgt.

4. Endoskop nach einem der Ansprüche 1 bis 3, **dadurch gekennzeichnet, daß** die lichtundurchlässige Schicht aus einem Material besteht, das einen Wärmeausdehnungskoeffizienten aufweist, der im Bereich der Wärmeausdehnungskoeffizienten der Materialien von Fenster (42, 72) und diesem umgebenden Element (44, 74) liegt.

5. Endoskop nach einem der Ansprüche 1 bis 4, **dadurch gekennzeichnet, daß** die lichtundurchlässige Schicht (46) auf der umfänglichen Mantelfläche (50) des Fensters (42) und/oder auf der radial inneren Mantelfläche (52) des diese umgebenden Elements (44) aufgebracht ist.

6. Endoskop nach einem der Ansprüche 1 bis 5, **dadurch gekennzeichnet, daß** die lichtundurchlässige Schicht aus einem metallischen Material besteht, das als vorgefertigte Überzugsschicht oder durch Sintern oder durch Aufdampfen auf die entsprechenden Flächen der Bauelemente aufgebracht ist.

7. Endoskop nach Anspruch 6, **dadurch gekennzeichnet, daß** die lichtundurchlässige Schicht als Rohrabschnitt (48) ausgebildet ist, der mit den Mantelflächen (50, 52) verschmolzen ist.

8. Endoskop nach Anspruch 6, **dadurch gekennzeichnet, daß** das Aufdampfen durch ein chemisches (CVD)- oder ein physikalisches (PVD)-Aufdampfverfahren erfolgt ist.

9. Endoskop nach einem der Ansprüche 2 bis 8, **dadurch gekennzeichnet, daß** eine radial äußere Mantelfläche (54) des Elements (44) mit einem Material beschichtet ist, das eine hermetisch dichte Verbindung mit der Innenseite (19) des distalen Endes des Außenrohrs (18) schafft.

10. Endoskop nach Anspruch 9, **dadurch gekennzeichnet, daß** die äußere Mantelfläche (54) des Elements (44) mit demselben Material beschichtet ist, das zur Verbindung zwischen Element (44) und dem Fenster (42) dient.

11. Endoskop nach einem der Ansprüche 2 bis 10, **dadurch gekennzeichnet, daß** die lichtundurchlässige Schicht auf seiten des Elements (44) als lichtreflektierende Schicht ausgebildet ist.

12. Endoskop nach einem der Ansprüche 2 bis 11, **dadurch gekennzeichnet, daß** die radial äußere Mantelfläche (54) des Elements (44) mit einer lichtreflektierenden Schicht versehen ist.

13. Endoskop nach einem der Ansprüche 1 bis 12, **dadurch gekennzeichnet, daß** zumindest eines der Materialien, ausgewählt aus der Gruppe bestehend aus der lichtdurchlässigen Schicht, das Material zur Verbindung der Bauelemente des Abschlußelementes untereinander, das Material zur Verbindung mit dem Außenrohr, die lichtreflektierende Schicht aus einem lötfähigen Material besteht.

14. Endoskop nach einem der Ansprüche 2 bis 13, **dadurch gekennzeichnet, daß** das Element (44) als Ring (45, 96, 106, 116) ausgebildet ist, in dem mittig das Fenster (42, 72, 125) passend sitzt.

15. Endoskop nach einem der Ansprüche 1 bis 14, **dadurch gekennzeichnet, daß** bei abgewinkelten Blickrichtungen das Abschlußelement (94, 104, 116) schräg zur Längsachse (119) des Außenrohrs (91, 101, 111) angeordnet ist.

16. Endoskop nach einem der Ansprüche 1 bis 15, **dadurch gekennzeichnet, daß** das Außenrohr als vom Endoskop (60, 140) abziehbares rohrförmiges (68, 146) Element ausgebildet ist, das über ein Schaftrohr (64, 142) schiebbar ist, welches das Innenrohr aufweist, und daß das Abschlußelement (70, 148) das abziehbare rohrförmige Element (68, 146) distalseitig hermetisch dicht abschließt.

17. Endoskop nach Anspruch 16, **dadurch gekennzeichnet, daß** das rohrförmige Element (68, 146) als steifes Rohr ausgebildet ist.

18. Endoskop nach Anspruch 16, **dadurch gekennzeichnet, daß** das rohrförmige Element als biegeschlaffer Schlauch ausgebildet ist.

19. Endoskop nach einem der Ansprüche 16 bis 18, **dadurch gekennzeichnet, daß** im abziehbaren rohrförmigen Element (146) die Lichtleiter (154) montiert sind.

20. Endoskop nach einem der Ansprüche 16 bis 19, **dadurch gekennzeichnet, daß** am Abschlußelement (70) Anlagen (82) vorgesehen sind, an die das distale Ende (83) des Schaftrohrs (64, 142) definiert positioniert zur Anlage kommt.

21. Endoskop nach einem der Ansprüche 1 bis 20, **dadurch gekennzeichnet, daß** ein Lichtanschluß (16) im proximalen Bereich des Endoskops (10), zu dem das proximale Ende der Lichtleiter (28) geführt ist, durch ein Lichtanschlußfenster (32) ebenfalls hermetisch dicht abgeschlossen ist.

22. Endoskop nach Anspruch 21, **dadurch gekennzeichnet, daß** das Lichtanschlußfenster (32) des Lichtanschlusses (16) gleichermaßen wie das Abschlußelement (40) hermetisch dicht angebracht ist.

23. Endoskop nach einem der Ansprüche 1 bis 22, **dadurch gekennzeichnet, daß** das Abschlußelement in dem Bereich, durch den das Beleuchtungslicht hindurchgeführt wird, als optisch wirksames Element, insbesondere als Diffusor oder Fokussierlinse ausgebildet ist.

24. Endoskop nach einem der Ansprüche 1 bis 23, **dadurch gekennzeichnet, daß** das Abschlußelement (134) in dem Bereich, durch den das Beleuchtungslicht hindurchgeführt wird, derart geformt ist, daß eine Lenkung des Beleuchtungslichts (138) aus der Leiterachse heraus erfolgt, wobei die Ausformung derart ist, daß an der Außenseite und/oder an der Innenseite des Abschlußelements (134) Abflachungen (137) vorhanden sind.

25. Endoskop nach einem der Ansprüche 1 bis 24, **dadurch gekennzeichnet, daß** das Fenster als optisch wirksames Element, insbesondere als Linse (125) und/oder Filter ausgebildet ist.

26. Endoskop nach einem der Ansprüche 1 bis 25, **dadurch gekennzeichnet, daß** das Abschlußelement (40) IR-Licht absorbierend ausgebildet ist.

27. Endoskop nach Anspruch 26, **dadurch gekennzeichnet, daß** die lichtundurchlässige Schicht (46), die das Fenster (42) umgibt, IR-Licht durchlässig ist.

28. Endoskop nach Anspruch 26 oder 27, **dadurch gekennzeichnet, daß** die Innenseite des Fensters (42) und/oder die Außenseite des Abschlußelementes (40) mit einer IR-Licht reflektierenden Schicht (47) beschichtet ist.

29. Endoskop nach einem der Ansprüche 26 bis 28, **dadurch gekennzeichnet, daß** die Innenseite des Abschlußelementes (40) in dem Bereich, in dem die Lichtleiter (28) münden, mit einer semipermeablen Schicht (49) beschichtet ist, die in Beleuchtungsrichtung lichtdurchlässig ist und in Gegenrichtung sperrt.

30. Verfahren zur Herstellung eines Abschlußelementes zum Verschließen des distalen Endes eines Endoskopes nach einem der Ansprüche 1 bis 29, **gekennzeichnet durch**:
a) Bereitstellen eines langen stabförmigen lichtdurchlässigen Materials mit dem Querschnitt des Fensters (42, 72, 125),
b) Bereitstellen eines langen rohrförmigen lichtdurchlässigen Materials, in dessen inneren Hohlraum das lange stabförmige Material etwa passend einführbar ist,
c) Vorsehen einer lichtundurchlässigen Schicht (46, 76, 98, 108, 118, 127, 236) zwischen Außenseite des stabförmigen Materials und Innenseite des Hohlraumes,
d) Ineinanderschieben von stabförmigem Material und rohrförmigem Material,
e) festes und dichtes Verbinden der Außenseite des stabförmigen Materials mit der Innenseite des Hohlraumes, und
f) Auftrennen des Zusammenbaus von stabförmigem Material und rohrförmigem Material in einzelne scheibenförmige Abschlußelemente (40, 70, 94, 104, 114, 124, 134, 148, 230).

31. Verfahren nach Anspruch 30, **dadurch gekennzeichnet, daß** in Schritt c) die Außenseite des stabförmigen Materials mit der lichtundurchlässigen Schicht (46, 76, 98, 108, 118, 127, 236) versehen wird.

## Claims

1. Endoscope having a head (12) at the proximal end, having an outer tube (18, 91, 101, 111, 121, 131, 222) joined to the head (12); having an inner tube (20, 62, 92, 102, 112, 122, 132, 142) received in the outer tube (18, 91, 101, 111, 121, 131, 222), in which optical components (58, 58', 144) are received; a window (42, 72, 125) made of a transparent material being arranged at the distal end of the inner tube (20, 62, 92, 102, 112, 122, 132, 142); and having light guides (28, 66, 93, 103, 113, 123, 133, 154, 242) that are received in an interstice (24) between inner tube (20, 62, 92, 102, 112, 122, 132, 142) and outer tube (18, 91, 101, 111, 121, 131, 222) and are guided axially to the distal end of the interstice (24, 152), wherein a closure element (40, 70, 94, 104, 114, 124, 134, 148, 230) made of a transparent material is provided, which closes off the distal end of the outer tube (18, 91, 101, 111, 121, 131, 222) in a hermetically sealed fashion at least in the region of the inner tube and of the interstice (24, 152) receiving the guide lights; the closure element (40, 70, 94, 104, 114, 124, 134, 148, 203) has in the region of the distal end of the inner tube the window (42, 72, 125) through which light can enter the inner tube, and the window (42, 72, 125) is surrounded circumferentially by an opaque layer (46, 76, 98, 108, 118, 127, 236) which shields the window (42, 72, 125) radially from the entry of illumination light that, coming from the light guides, is guided through the closure element and emerges at the distal end, **characterized in that** the closure element (40, 70, 94, 104, 114, 124, 134, 148, 230) is made of merely two components, i.e. the central window (42, 72, 125) which is inserted into the closure element (40, 70, 94, 114, 124, 134, 148, 230) and connected to it via the circumferential opaque layer (46, 76, 98, 108, 118, 127, 236) only, and **in that** the closure element (40, 70, 94, 104, 124, 134, 148, 230) is connected with the outer tube (18, 91, 101, 111, 121, 131, 222) via its outer circumferential edge only.

2. Endoscope of claim 1, **characterized in that** the closure element (40, 70, 94, 104, 114, 130) is made up of the window (42, 72, 125), an opaque layer (46, 76, 98, 108, 118, 127, 236) covering its circumferential enveloping surface (50) and an element (44, 74) surrounding it.

3. Endoscope of claims 1 or 2, **characterized in that** the opaque layer is configured in such a way that it provides not only shielding but also a hermetically sealed join between the window (42, 72) and the element (44, 74) surrounding it.

4. Endoscope of anyone of claims 1 through 3, **characterized in that** the opaque layer is made of a material which has a coefficient of thermal expansion that lies in the range of the coefficience of thermal expansions of the materials of the window (42, 72) and the element (44, 74) surrounding it.

5. Endoscope of anyone of claims 1 through 4, **characterized in that** the opaque layer (46) is applied on the circumferential enveloping surface (50) of the window (42) and/or on the radially inner enveloping surface (52) of the annular element (44) surrounding it.

6. Endoscope of anyone of claims 1 through 5, **characterized in that** the opaque layer is made of a metallic material that is applied onto the corresponding surfaces of the components as a prefabricated coating layer or by sintering or by vacuum deposition.

7. Endoscope of claim 1, **characterized in that** the opaque layer is configured as a tubular segment (48) that is fused to the enveloping surfaces (50, 52).

8. Endoscope of claim 6, **characterized in that** the vacuum deposition is performed by a chemical (CVD) or a physical (PVD) vapor deposition method.

9. Endoscope of anyone of claims 2 through 8, **characterized in that** a radially outer enveloping surface (54) of the element (44) is coated with a material that creates a hermetically sealed join with the inner side (19) of the distal end of the outer tube (18).

10. Endoscope of claim 9, **characterized in that** the outer enveloping surface (54) of the element (44) is coated with the same material that provides the join between the element (44) and the window (42).

11. Endoscope of anyone of claims 2 through 10, **characterized in that** the opaque layer is configured on the element (44) as a light-reflecting layer.

12. Endoscope of anyone of claims 2 through 11, **characterized in that** the radially outer enveloping surface (54) of the element (44) is provided with a light-reflecting layer.

13. Endoscope of anyone of claims 1 through 12, **characterized in that** at least one of the materials selected from the group consisting of the opaque layer, the material for joining the components of the closure elements to one another, the material for connecting with the outer tube, the light reflecting layer is made of a solderable material.

14. Endoscope of anyone of claims 2 through 13, **characterized in that** the element (44) is configured as a ring (54, 96, 106, 116) in which the window (42, 72, 125) sits in centeredly fitted fashion.

15. Endoscope of anyone of claims 1 through 14, **characterized in that** when the viewing directions are angled, the closure element (94, 104, 116) is arranged obliquely with respect to the longitudinal axis (119) of the outer tube (91, 101, 111).

16. Endoscope of anyone of claims 1 through 15, **characterized in that** the outer tube is configured as a tubular (68, 146) element which can be pulled off from the endoscope (60, 140) and is slidable over a shaft tube (64, 142) which bears the inner tube; and **in that** the closure element (70, 148) closes off the pull-off tubular element (68, 146) at the distal end in a hermetically sealed fashion.

17. Endoscope of claim 16, **characterized in that** the tubular element (68, 146) is configured as a rigid tube.

18. Endoscope of claim 16, **characterized in that** the tubular element is configured as a flexurally limp hose.

19. Endoscope of anyone of claims 16 through 18, **characterized in that** the light guides (154) are installed in the pull-off tubular element (146).

20. Endoscope of anyone of claims 16 through 19, **characterized in that** supports (82) are provided on the closure element (70), against which supports (82) the distal end (83) of the shaft tube (64, 122) comes into contact in a defined position.

21. Endoscope of anyone of claims 1 through 20, **characterized in that** a light connector (16) in the proximal region of the endoscope (10), to which the proximal end of the light guides (28) is guided, is also closed off in a hermetically sealed fashion by a light connector window (32).

22. Endoscope of claim 21, **characterized in that** the. light connector window (32) of the light connector (16) is mounted in hermetically sealed fashion in the same way as the closure element (40).

23. Endoscope of anyone of claims 1 through 22, **characterized in that** the closure element is configured, in the region through which the illumination light is passed, as an optically effective element, in particular as a diffusor or a focusing lens.

24. Endoscope of anyone of claims 1 through 23, **characterized in that** the closure element (134) is shaped, in the region through which the illumination light is passed, in such a way that a deflection of the illumination light (138) out of the guide axis is accomplished, wherein said shaping is such that flattened areas (137) are present on the outer side and/or on the inner side of the closure element (134).

25. Endoscope of anyone of claims 1 through 24, **characterized in that** the window is configured as an optically effective element, in particular as a lens (125) and/or a filter.

26. Endoscope of anyone of claims 1 through 25, **characterized in that** the closure element (40) is configured to absorb IR-light.

27. Endoscope of claim 26, **characterized in that** the opaque layer (46), which surrounds the window (42), is transparent to IR-light.

28. Endoscope of claims 26 or 27, **characterized in that** the inner side of the window (42) and/or the outer side of the closure element (40) is coated with a layer (47) that reflects IR-light.

29. Endoscope of anyone of claims 26 through 28, **characterized in that** the inner side of the closure element (40) is coated, in the region in which the light guides (28) terminate, with a semi-permeable layer (49) that is transparent in the illumination direction and blocks light in the opposite direction.

30. Method for manufacturing a closure element for closing off the distal end of an endoscope as defined in anyone of claims 1 through 29, **characterized by** the steps of
a) providing a long, rod-shaped, transparent material having a cross section of the window (42, 72, 125);
b) providing a long, tubular, transparent material into whose inner cavity the rod-shaped material can be introduced so as to fit approximately;
c) providing an opaque layer (46, 76, 98, 108, 118, 127, 236) between the outer side of the rod-shaped material and the inner side of the cavity;
d) sliding the rod-shaped material and the tubular material into one another;
e) joining the outer side of the rod-shaped material in a permanent and sealed fashion to the inner side of the cavity; and
f) cutting the assembly of rod-shaped material and tubular material into individual disk-shaped closure elements (40, 70, 94, 104, 114, 124, 134, 148, 230).

31. Method of claim 30, **characterized in that**, that in step c) the outer side of the rod-shaped material is equipped with the opaque layer (46, 76, 98, 108, 118, 127, 236).

## Revendications

1. Endoscope, avec une tête (12) à l'extrémité proximale, avec un tube extérieur (18, 91, 101, 111, 121, 131, 222) relié à la tête (12), avec un tube intérieur (20, 62, 92, 102, 112, 122, 132, 142) logé dans le tube extérieur (18, 91, 101, 111, 121, 131, 222), tube intérieur dans lequel sont logés des éléments optiques (58, 58', 144), une fenêtre (42, 72, 125) en matériau transparent étant placée à l'extrémité distale du tube intérieur (20, 62, 92, 102, 112, 122, 132, 142), ainsi qu'avec des conducteurs optiques (28, 66, 93, 103, 113, 123, 133, 154, 242) qui sont logés dans un interstice (24) entre le tube intérieur (20, 62, 92, 102, 112, 122, 132, 142) et le tube extérieur (18, 91, 101, 111, 121, 131, 222) et sont dirigés axialement jusqu'à l'extrémité distale de l'interstice (24, 152), un élément de fermeture (40, 70, 94, 104, 114, 124, 134, 148, 230) en matériau transparent étant prévu, qui ferme hermétiquement l'extrémité distale du tube extérieur (18, 91, 101, 111, 121, 131, 222) au moins au niveau du tube intérieur et de l'interstice (24, 152) recevant les conducteurs optiques, dans lequel l'élément de fermeture (40, 70, 94, 104, 114, 124, 134, 148, 230) présente au niveau de l'extrémité distale du tube intérieur la fenêtre (42, 72, 125) par laquelle de la lumière peut pénétrer dans le tube intérieur, et la fenêtre (42, 72, 125) est entourée sur son pourtour par une couche opaque à la lumière (46, 76, 98, 108, 118, 127, 136) qui protège la fenêtre (42, 72, 125) radialement contre l'entrée d'une lumière d'éclairage qui, provenant des conducteurs optiques, est envoyée à travers l'élément de fermeture et sort au niveau de l'extrémité distale, **caractérisé en ce que** l'élément de fermeture (40, 70, 94, 104, 114, 124, 134, 148, 230) est constitué de seulement deux éléments, à savoir la fenêtre centrale (42, 72, 125), qui est insérée dans l'élément de fermeture (40, 70, 94, 104, 114, 124, 134, 148, 230) et n'est reliée à celui-ci que par la couche opaque périphérique (46, 76, 98, 108, 118, 127, 136), et **en ce que** l'élément de fermeture (40, 70, 94, 104, 114, 124, 134, 148, 230) n'est relié au tube extérieur (18, 91, 101, 111, 121, 131, 222) que par son bord périphérique extérieur.

2. Endoscope selon la revendication 1, **caractérisé en ce que** l'élément de fermeture (40, 70, 94, 104, 114, 130) est composé de la fenêtre (42, 72, 125), d'une couche opaque (46, 76, 98, 108, 118, 127, 136) recouvrant la surface d'enveloppe périphérique de celle-ci, et d'un élément (44, 74) l'entourant.

3. Endoscope selon la revendication 1 ou 2, **caractérisé en ce que** la couche opaque est conformée de telle manière que celle-ci, outre la protection, assure également une liaison hermétique entre la fenêtre (42, 72) et l'élément (44, 74) entourant cette dernière.

4. Endoscope selon l'une des revendications 1 à 3, **caractérisé en ce que** la couche opaque est composée d'un matériau qui présente un coefficient de dilatation thermique qui est de l'ordre des coefficients de dilatation thermique des matériaux de la fenêtre (42, 72) et de l'élément (44, 74) entourant cette dernière.

5. Endoscope selon l'une des revendications 1 à 4, **caractérisé en ce que** la couche opaque (46) est appliquée sur la surface d'enveloppe périphérique (50) de la fenêtre (42) et/ou sur la surface d'enveloppe radialement intérieure (52) de l'élément (44) l'entourant.

6. Endoscope selon l'une des revendications 1 à 5, **caractérisé en ce que** la couche opaque est composée d'un matériau métallique qui est appliqué sous forme de couche de revêtement préfabriquée ou par frittage ou par vaporisation sur les surfaces correspondantes des éléments.

7. Endoscope selon la revendication 6, **caractérisé en ce que** la couche opaque est conformée en section de tube (48) qui est fusionnée aux surfaces d'enveloppe (50, 52).

8. Endoscope selon la revendication 6, **caractérisé en ce que** la vaporisation s'effectue par un procédé de vaporisation chimique (CVD) ou physique (PVD).

9. Endoscope selon l'une des revendications 2 à 8, **caractérisé en ce qu'**une surface d'enveloppe radialement extérieure (54) de l'élément (44) est revêtue d'un matériau qui crée une liaison hermétique avec la face intérieure (19) de l'extrémité distale du tube extérieur (18).

10. Endoscope selon la revendication 9, **caractérisé en ce que** la surface d'enveloppe extérieure (54) de l'élément (44) est revêtue du même matériau que celui qui sert à la liaison entre l'élément (44) et la fenêtre (42).

11. Endoscope selon l'une des revendications 2 à 10, **caractérisé en ce que** la couche opaque est conformée en couche réfléchissant la lumière du côté de l'élément (44).

12. Endoscope selon l'une des revendications 2 à 11, **caractérisé en ce que** la surface d'enveloppe radialement extérieure (54) de l'élément (44) est munie d'une couche réfléchissant la lumière.

13. Endoscope selon l'une des revendications 1 à 12, **caractérisé en ce qu'**au moins l'un des matériaux, choisi dans le groupe composé de la couche transparente, du matériau pour lier entre eux les composants de l'élément de fermeture, du matériau pour la liaison avec le tube extérieur, de la couche réfléchissante, est composé d'un matériau soudable.

14. Endoscope selon l'une des revendications 2 à 13, **caractérisé en ce que** l'élément (44) est conformé en anneau (45, 96, 106, 116) dans lequel la fenêtre (42, 72, 125) est ajustée en position centrale.

15. Endoscope selon l'une des revendications 1 à 14, **caractérisé en ce que** pour des directions d'observation obliques, l'élément de raccordement (94, 104, 116) est disposé à l'oblique par rapport à l'axe longitudinal (119) du tube extérieur (91, 101, 111).

16. Endoscope selon l'une des revendications 1 à 15, **caractérisé en ce que** le tube extérieur est conformé en élément tubulaire (68, 146) extractible depuis l'endoscope (60, 140), qui peut être déplacé par l'intermédiaire d'une tige tubulaire (64, 142) qui présente le tube intérieur, et **en ce que** l'élément de fermeture (70, 148) ferme hermétiquement l'élément tubulaire extractible (68, 146) du côté distal.

17. Endoscope selon la revendication 16, **caractérisé en ce que** l'élément tubulaire (68, 146) est conformé en tube rigide.

18. Endoscope selon la revendication 16, **caractérisé en ce que** l'élément tubulaire est conformé en tuyau souple.

19. Endoscope selon l'une des revendications 16 à 18, **caractérisé en ce que** les conducteurs optiques (154) sont montés dans l'élément tubulaire extractible (146).

20. Endoscope selon l'une des revendications 16 à 19, **caractérisé en ce que** sur l'élément de fermeture (70) sont prévus des appuis (82) contre lesquels l'extrémité distale (83) de la tige tubulaire (64, 142) vient s'appuyer dans une position définie.

21. Endoscope selon l'une des revendications 1 à 20, **caractérisé en ce qu'**un raccord optique (16) dans la partie proximale de l'endoscope (10), vers laquelle est dirigée l'extrémité proximale des conducteurs optiques (28), est également fermé hermétiquement par une fenêtre de raccord optique (32).

22. Endoscope selon la revendication 21, **caractérisé en ce que** la fenêtre de raccord optique (32) du raccord optique (16) est appliquée de façon hermétique de la même manière que l'élément de fermeture (40).

23. Endoscope selon l'une des revendications 1 à 22, **caractérisé en ce que**, dans la zone à travers laquelle passe la lumière d'éclairage, l'élément de fermeture est conformé en élément à effet optique, en particulier en diffuseur ou en lentille de focalisation.

24. Endoscope selon l'une des revendications 1 à 23, **caractérisé en ce que**, dans la zone à travers laquelle passe la lumière d'éclairage, l'élément de fermeture (134) est formé de telle manière qu'il se produit une déviation de la lumière d'éclairage (138) depuis l'axe du conducteur, la configuration étant telle que des méplats (137) sont présents sur la face extérieure et/ou sur la face intérieure de l'élément de fermeture (134).

25. Endoscope selon l'une des revendications 1 à 24, **caractérisé en ce que** la fenêtre est conformée en élément à effet optique, en particulier en lentille (125) et/ou en filtre.

26. Endoscope selon l'une des revendications 1 à 25, **caractérisé en ce que** l'élément de fermeture (40) est réalisé absorbant pour la lumière IR.

27. Endoscope selon la revendication 26, **caractérisé en ce que** la couche opaque (46) qui entoure la fenêtre (42) est transparente pour la lumière IR.

28. Endoscope selon la revendication 26 ou 27, **caractérisé en ce que** la face intérieure de la fenêtre (42) et/ou la face extérieure de l'élément de fermeture (40) est revêtue d'une couche (47) réfléchissant la lumière IR.

29. Endoscope selon l'une des revendications 26 à 28, **caractérisé en ce que**, dans la zone dans laquelle débouchent les conducteurs optiques (28), la face intérieure de l'élément de fermeture (40) est revêtue d'une couche semi-perméable (49), qui est transparente dans la direction d'éclairage et opaque dans la direction inverse.

30. Procédé de fabrication d'un élément de fermeture pour fermer l'extrémité distale d'un endoscope selon l'une des revendications 1 à 29, **caractérisé par** :
a) la fourniture d'un matériau transparent en forme de bâtonnet allongé ayant la section transversale de la fenêtre (42, 72, 125),
b) la fourniture d'un matériau transparent tubulaire allongé, dans la cavité intérieure duquel un matériau en bâtonnet allongé peut être introduit avec ajustement approximatif,
c) le placement d'une couche opaque (46, 76, 98, 108, 118, 127, 136) entre la face extérieure du matériau en forme de bâtonnet et la face intérieure de la cavité,
d) l'enfoncement l'un dans l'autre du matériau en forme de bâtonnet et du matériau tubulaire,
e) la liaison rigide et hermétique de la face extérieure du matériau en forme de bâtonnent avec la face intérieure de la cavité, et
f) la séparation de l'assemblage composé du matériau en forme de bâtonnet et du matériau tubulaire en différents éléments de fermeture (40, 70, 94, 104, 114, 124, 134, 148, 230) en forme de plaque.

31. Procédé selon la revendication 30, **caractérisé en ce qu'**à l'étape c), la face extérieure du matériau en forme de bâtonnet est munie de la couche opaque (46, 76, 98, 108, 118, 127, 136).
